# EUROPÄISCHE PATENTANMELDUNG

(11) **EP 0 678 304 A1**
(43) Veröffentlichungstag der Anmeldung: **25.10.1995**
(21) Anmeldenummer: 94115803.2
(22) Anmeldetag: 07.10.1994
(51) Int. Cl.: A61M 16/00

(54) **Steuerung für den Durchflussregler in einem Sauerstofftherapiegerät**

(30) Priorität: 18.04.1994 DE 9406407 U
(71) Anmelder: Schneider, Peter, D-56759 Laubach (DE)
(72) Erfinder: Schneider, Peter, D-56759 Laubach (DE)
(74) Vertreter: Grommes, Karl F., Dr.

(57) **Zusammenfassung**

Bei dem Erfindungsgegenstand handelt es sich um ein Sauerstoffgerät, daß über eine spezeille Steuerung nur während der Einatmung Sauerstoff zuführt. Dabei wird die Gegebenheit genutzt, daß sich während eines Atemzyklus unterschiedliche elektrische Spannungen von der Atemmuskulatur ableiten lassen, die ein typisches Profil aufweisen: einen Anstieg (4) der elektrischen Spannung während der Einatmung und einen Abfall (5) der elektrischen Spannung während der passiven Ausatmung.

In einer elektronischen Meß- und Steuereinheit (3) werden die mittels Elektroden (1) und Kabel (2) abgeleiteten Spannungen registriert und elektronisch verarbeitet und zur Steuerung des Durchflußreglers (fi) herangezogen. Die Arbeit der Atemmuskulatur bewirkt bei der Einatmung einen Anstieg der elektr. Spannung (4), die den Durchflußregler öffnet. Während der passiven Ausatmung fällt die elektr. Spannung wieder ab (5), der Durchflußregler beendet die Sauerstoffzufuhr. Aufgrund dieser Steuerung lassen sich im Mittel etwa 2/3 der Sauerstoffmenge einsparen.

## Beschreibung

Die Erfindung bezieht sich auf die Steuerung eines Sauerstoffgerätes, die veranlaßt, daß bei der Einatmung Sauerstoff verabreicht und bei der Ausatmung die Sauerstoffzufuhr beendet wird. Dabei werden mittels Elektroden elektrische Spannungen abgeleitet, die bei der Arbeit der Atemmuskulatur entstehen, von einer elektronischen Meß- und Steuereinheit verarbeitet und zum Öffnen und Schließen eines Durchflußreglers genutzt.

Sauerstoffgeräte ermöglichen die Anreicherung der Atemluft mit zusätzlichem Sauerstoff. Dabei wird an einer Armatur mittels eines Stellrades ein bestimmter Gasfluß eingestellt, der dem Patienten kontinuierlich Sauerstoff während des gesamten Atemzyklus zugeführt. Ein Atemzyklus setzt sich zusammen aus Einatmung, Ausatmung und einer evtl. kurzen Pause, das durchschnittliche Verhältnis ist etwa 1 : 2. Der zugeführte Sauerstoff während der Ausatemphase kann vom Patienten nicht genutzt werden und wird somit verschwendet.

Hier will die Erfindung Abhilfe schaffen. Die Erfindung, wie sie in den Ansprüchen gekennzeichnet ist, löst die Aufgabe, die während der Ausatmung unnötig verabreichte Sauerstoffmenge einzusparen. Aufgrund der Tatsache, daß bei der Arbeit der Atemmuskulatur elektrische Potentiale freigesetzt werden, steuert der Patient die Zufuhrzeit von Sauerstoff während eines Atemzyklus selbst.Während der Einatmung steigt die elektrische Spannung an, während der Ausatmung fällt sie wieder ab. Hierbei entsteht ein typisches Spannungsprofil und zwar in Form eines aufsteigenden und eines absteigenden Abschnittes. Diese sich ständig ändernde elektr.

Spannung wird mittels Elektroden und eines Kabels zu einer Meß- und Steuereinheit abgeleitet und dort zum Öffnen und Schließen eines Durchflußreglers genutzt In der Meß- und Steuereinheit wird die zugeleitete elektrische Spannung elektronisch ausgewertet, steigt die elektr. Spannung an, dient dieses als Signal, den Durchflußregler zu öffnen, fällt die elektr. Spannung ab, dient dies als Signal, den Durchflußregler zu schließen.

Die Vorteile der Erfindung sind darin zu sehen, daß Sauerstoff nur dann appliziert wird, wenn er auch tatsächlich medizinisch wirksam sein kann. Aufgrund der Erfindung können im Mittel ca. 2/3 Sauerstoff eingespart werden, was zu einer erheblichen Kosteneinsparung führt.

Im folgenden wird die Erfindung anhand von Zeichnungen näher erläutert. Es zeigt
Figur 1 die Erfindung in chematischer Darstellung und
Figur 2 hintereinanderfolgend drei elektrische Spannungsprofile (Analog zu drei Atemzyklen) sowie darunter die jeweiligen Schaltmomente.

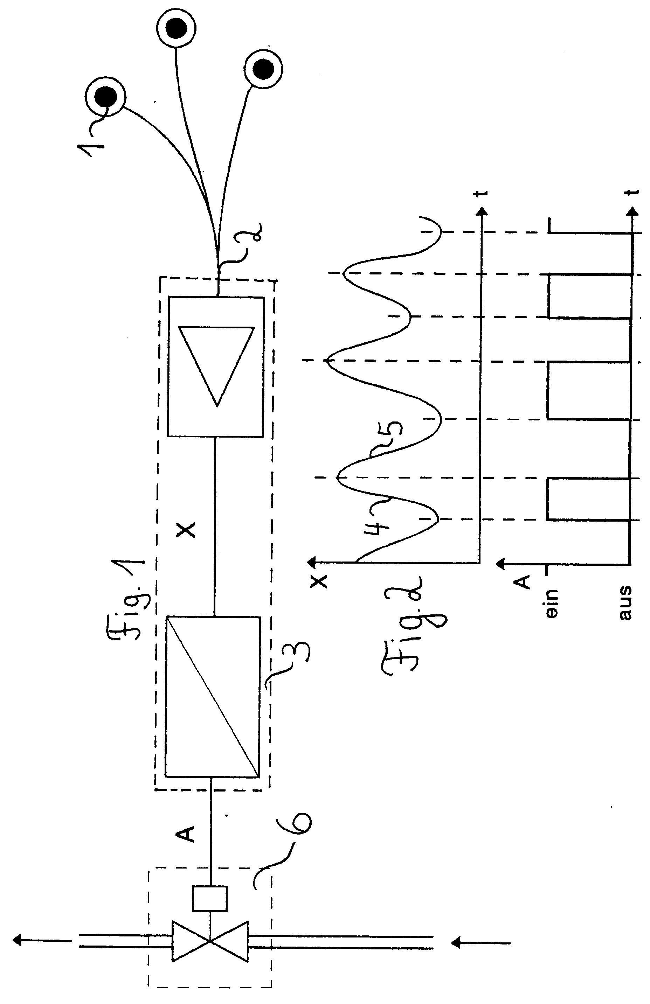

Die auf dem Brustkorb des Patienten applizierten Elektroden (1) nehmen das dort herrschende elektrische Spannungpotential auf und leiten es über ein Kabel (2) zu einer elektronischen Meß- und Steuereinheit (3), die dieses kontinuierlich registriert evtl. verstärkt, vermißt und dieses in Schaltsignale umwandelt. Das sich dabei zeigende elektrische Spannungsprofil weist einen aufsteigenden (4) und einen absteigenden (5) Schenkel auf. Spannunsanstieg und Spannungsabstieg werden von einer elektronischen Meß- und Steuereinheit (3) zum Steuern des Durchflußreglers (6) genutzt.

## Patentansprüche

1. Sauerstoffgerät mit integrierter Steuerung für das Öffnen und Schließen des Durchflußreglers, bestehend aus einem ableitenden Teil in Form von Elektroden (1) und Kabel (2), einer Meß- und Steuereinheit (3) sowie einem Gasdurchflußregler (6), dadurch gekennzeichnet, daß aufgrund elektrischer Impulse (die durch die Arbeit der Atemmuskulatur entstehen) der Durchflußregler (6) für Sauerstoff intermittierend öffnet und schließt, wobei ein elektr. Spannungsanstieg (4) als Schaltsignal für die Öffnung und ein elektr. Spannungsabfall (5) als Schaltsignal für die Schließung des Durchflußreglers (6) dient. Der Durchflußregler wird dabei von einer Meß- und Steuereinheit (3) gesteuert, der die abgeleiteten elektrischen Spannungen registriert, auswertet und in Schaltsignale umwandelt und so für die gewünschte Stellung des Durchflußreglers sorgt.

2. Sauerstoffgerät nach Anspruch 1, bei dem sich die zu verabreichende Sauerstoffmenge pro Atemzug begrenzen läßt. Hierbei wird ab Zeitpunkt des Beginns eines elektrischen Spannungsanstieges (4) eine vorher bestimmte Sauerstoffmenge (z.B. 200ml) verabreicht. Der Durchflußregler schließt dann nach Verabreichung dieser Sauerstoffmenge, obwohl die Einatmung noch fortdauert. Die Schaltmomente liegen dann innerhalb eines Atemzyklus für das Öffnen des Durchflußreglers am Beginn des Spannungsanstieges und für das Schließen des Durchflußreglers nach erfolgter Zufuhr der vorgewählten Sauerstoffmenge.

3. Sauerstoffgerät nach Anspruch 1, bei dem sich die Meß- und Steuereinheit (3) nicht im oder am Sauerstoffgerät selbst befindet, sondern in einem EKG-Monitor. Hierbei werden die elektrischen Ströme des Herzens und der Atemmuskulatur über ein gemeinsames Kabel zu einem EKG-Monitor geleitet und dort mittels eines Frequenzfilters getrennt. Die herausgefilterten elektrischen Ströme der Atemmuskulatur werden dann im EKG-Monitor mittels der Meß- und Steuereinheit elektronisch zu Schaltsignalen verarbeitet, die dann über Kabel den Durchflußregler im Sauerstoffgerät zu der gewünschten Stellung bringen.

4. Sauerstoffgerät nach Anspruch 1 und 3, bei dem sich Meß- und Steuereinheit sowie Durchflußregler gemeinsam im Sauerstoffgerät befinden, jedoch die elektr. Ströme der Atemmuskulatur gemeinsam mit den Herzströmen zuerst an einen EKG-Monitor geführt werden, dort wieder von diesen mittels Frequenzfilterung getrennt und der Meß- und Steuereinheit an oder im Sauerstoffgerät zur elektronischen Verarbeitung mittels Kabel zugeleitet werden.
